# EUROPEAN PATENT APPLICATION

(11) **EP 4 813 973 A1**
(43) Date of publication of application: **30.09.2026**
(21) Application number: 24893464.8
(22) Date of filing: 20.11.2024
(51) Int. Cl.: C07D 211/72, A61K 31/44, A61P 7/02

(54) **BENZENESULFONATE CONTAINING PIPERIDINE RING COMPOUND, PREPARATION METHOD THEREFOR, AND USE THEREOF**

(30) Priority: 24.11.2023 CN 202311584010
(71) Applicant: Shanghai Curegene Pharmaceutical Co., Ltd., Shanghai 200135 (CN)
(72) Inventor: HE, Gongxin, Shanghai 200135 (CN); HOU, Kai, Shanghai 200135 (CN); TANG, Xiubo, Shanghai 200135 (CN); FAN, Wenyuan, Shanghai 200135 (CN)
(74) Representative: Vossius & Partner Patentanwälte Rechtsanwälte mbB
(86) International application number: PCT/CN2024/133212
(87) International publication number: WO 2025/108307

(57) **Abstract**

Disclosed in the present invention are a benzenesulfonate containing a piperidine ring compound, a preparation method therefor, and a use thereof. Specifically, provided in the present invention is compound 2. Compound 2 can exist stably, and by means of a step of salt preparation, the content of the isomer impurity shown in formula 1' can be significantly reduced.

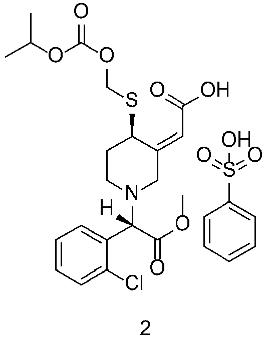

## Description

The present application claims priority to Chinese Patent Application No. CN202311584010X filed on November 24, 2023, which is incorporated herein by reference in its entirety.

### TECHNICAL FIELD

The present disclosure relates to benzenesulfonate of a piperidine ring-containing compound, a preparation method therefor, and use thereof.

### BACKGROUND

Compound 1 is a compound with anti-platelet activity. The compound can, under the action of a hydrolase *in vivo*, generate an active metabolite of clopidogrel, thereby further irreversibly inhibiting platelet coagulation activity.

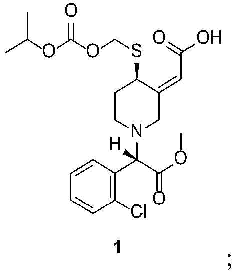

The crude product of compound 1 obtained by the preparation process in the prior art contains an isomer impurity represented by formula 1' (impurity 1' for short). Impurity 1' cannot be removed by conventional purification means such as recrystallization and column chromatography, and compound 1 cannot form a stable salt with a conventional acid or base, such as hydrochloric acid, sulfuric acid, and sodium hydroxide.

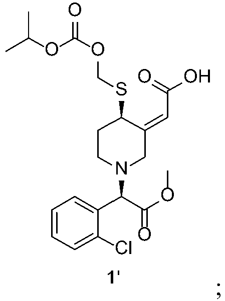

Characterization data for impurity 1': **LCMS:** [M+ H] ⁺ = 472.1; **¹H NMR:** (400 MHz, DMSO-d6) δ = 12.52 (br s, 1H), 7.53 - 7.44 (m, 2H), 7.43 - 7.34 (m, 2H), 5.66 (s, 1H), 5.34 (br d, J = 2.8 Hz, 1H), 5.27 (d, J = 12.1 Hz, 1H), 5.10 (d, J = 12.1 Hz, 1H), 4.82 - 4.70 (m, 2H), 3.65 (s, 3H), 3.45 (d, J = 12.1 Hz, 1H), 3.10 (br d, J = 12.5 Hz, 1H), 2.75 - 2.57 (m, 2H), 2.11 - 1.98 (m, 1H), 1.82 (br dd, J = 1.8, 14.2 Hz, 1H), 1.22 (dd, J = 2.8, 6.2 Hz, 6H).

### SUMMARY

The technical problem to be solved in the present disclosure is to overcome the defect in the prior art that an isomer impurity represented by formula 1' is difficult to remove, in particular, to reduce the content of impurity 1' from about 2% to a lower level, thereby providing benzenesulfonate of a piperidine ring-containing compound, a preparation method therefor, and use thereof. It has been unexpectedly found in the present disclosure that compound 1 can form a salt with benzenesulfonic acid, and by preparing the salt form, the isomer impurity described above can be removed. Through this step, the content of the isomer impurity represented by formula 1' can be significantly reduced.

The present disclosure mainly solves the technical problem described above through the following technical solutions.

The present disclosure provides compound 2:

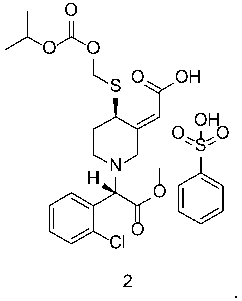

The present disclosure further provides a preparation method for compound 2 described above, which comprises the following step: mixing compound 1 with benzenesulfonic acid in a solvent, wherein the solvent is an ether solvent, a mixed solvent of an ether solvent and ethanol, an ester solvent, or an alkane solvent;

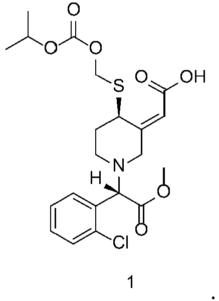

In a certain embodiment, the method comprises the following step: mixing solution 1 containing compound 1 with solution 2 containing benzenesulfonic acid for a reaction, wherein a solvent of solution 1 is an ether solvent, a mixed solvent of an ether solvent and ethanol, or an ester solvent; a solvent of solution 2 is an ether solvent or an alkane solvent; the ether solvent may be isopropyl ether; the ester solvent may be ethyl acetate; the alkane solvent may be n-heptane.

In the preparation method for compound 2, a purity of compound 1 may be 90%-99.6%, such as 94.6% or 97.5%.

In a certain embodiment, compound 1 is a crude product of compound 1, and the crude product of compound 1 is a mixture of compound 1 and an isomer impurity represented by formula 1'; a mass ratio of compound 1 to the isomer impurity may be (10-100):1, such as 23.47:1 or 42.03:1;

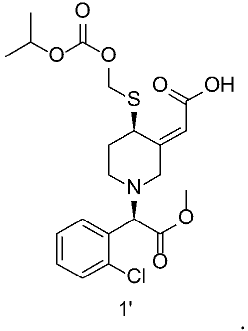

In a certain embodiment, in the preparation method for compound 2, when the solvent is a mixed solvent of an ether solvent and ethanol, a volume ratio of the ether solvent to ethanol is (1-10):1, such as 5:1.

In a certain embodiment, in the preparation method for compound 2, a mass-to-volume ratio of compound 1 to the solvent of solution 1 is 1:(1-10) g/mL, such as 1:2.3 g/mL, 1:4 g/mL, or 1:6 g/mL.

In a certain embodiment, in the preparation method for compound 2, a molar ratio of benzenesulfonic acid to compound 1 may be (0.3-1):1, such as 0.5:1, 0.98:1, or 1:1.

In a certain embodiment, in the preparation method for compound 2, a mass-to-volume ratio of benzenesulfonic acid to the solvent of solution 2 is 1:(0.1-10) g/mL, such as 1:0.15 g/mL, 1:0.7 g/mL, or 1:10 g/mL.

In a certain embodiment, the preparation method for compound 2 further comprises a step of mixing with solvent 3, wherein solvent 3 is a mixed solvent of an ether solvent and acetonitrile, or an alkane solvent, wherein the ether solvent is isopropyl ether, and the alkane solvent is n-heptane; when solvent 3 is an ether solvent and acetonitrile, a volume ratio of the ether solvent to acetonitrile may be (13-20):1, such as 15:1.

In a certain embodiment, in the preparation method for compound 2, a mass-to-volume ratio of compound 1 to solvent 3 is 1:(3-45) g/mL, such as 1:6 g/mL, 1:17.85 g/mL, or 1:27 g/mL.

In a certain embodiment, the preparation method for compound 2 may further comprise adding solution 2 containing benzenesulfonic acid to solution 1 containing compound 1, and an addition rate may be 1-10 V/h, such as 4 V/h.

In a certain embodiment, the preparation method for compound 2 may further comprise adding solvent 3 to a mixed solution of solution 1 containing compound 1 and solution 2 containing benzenesulfonic acid, and an addition rate may be 0.1-3 V/h, such as 1.5 V/h.

In a certain embodiment, in the preparation method for compound 2, a mixing temperature is a conventional temperature in the art for such reactions, such as 25 °C.

In a certain embodiment, in the preparation method for compound 2, a mixing reaction time is 5-100 h, such as 9.3 h, 22 h, or 73 h.

In a certain embodiment, the preparation method for compound 2 comprises scheme A or scheme B:

Scheme A: mixing solution 1 containing compound 1 with solution 2 containing benzenesulfonic acid for a reaction, wherein a solvent of solution 1 is a mixed solvent of isopropyl ether and ethanol (e.g., a volume ratio of isopropyl ether to ethanol is 5:1), and a solvent of solution 2 is isopropyl ether; further, after the mixing and reaction, a step of mixing with solvent 3 is further comprised, wherein solvent 3 is a mixed solution of isopropyl ether and acetonitrile (e.g., a volume ratio of isopropyl ether to ethanol is 15:1);

Scheme B: mixing solution 1 containing compound 1 with solution 2 containing benzenesulfonic acid for a reaction, wherein a solvent of solution 1 is ethyl acetate; a solvent of solution 2 is n-heptane.

In a certain embodiment, solution 1 containing compound 1 is mixed with solution 2 containing benzenesulfonic acid for a reaction, wherein a solvent of solution 1 is an ether solvent, a mixed solvent of an ether solvent and ethanol, or an ester solvent; a solvent of solution 2 is an ether solvent or an alkane solvent; the ether solvent is isopropyl ether; the ester solvent is ethyl acetate; the alkane solvent is n-heptane; a purity of compound 1 is 90%-99.6%; compound 1 is a crude product of compound 1, and the crude product of compound 1 is a mixture of compound 1 and an isomer impurity represented by formula 1'; a mass ratio of compound 1 to the isomer impurity is (10-100):1; a mass-to-volume ratio of compound 1 to the solvent of solution 1 is 1:(1-10) g/mL; a molar ratio of benzenesulfonic acid to compound 1 is (0.3-1):1; a mass-to-volume ratio of benzenesulfonic acid to the solvent of solution 2 is 1:(0.1-10) g/mL; further, a step of mixing with solvent 3 may further be comprised, wherein solvent 3 is a mixed solvent of an ether solvent and acetonitrile or an alkane solvent, wherein the ether solvent is isopropyl ether; the alkane solvent is n-heptane; a mass-to-volume ratio of compound 1 to solvent 3 is 1:(3-45) g/mL.

A purity of compound 2 prepared by the preparation method for compound 2 is 98.5% or higher, such as 98.9% or 99%.

A content of impurity 1' in a product prepared by the preparation method for compound 2 is less than 1%, such as 0.8% or less than 0.1%.

The present disclosure further provides use of compound 2 described above in the preparation of compound 1;

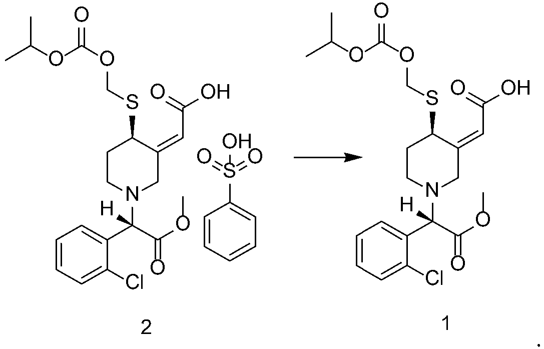

The present disclosure further provides a purification method for compound 1, which comprises the following steps: 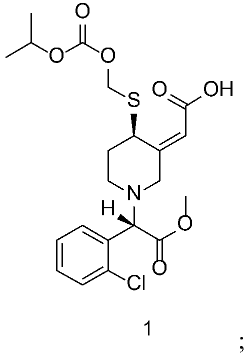
S1: the preparation method for compound 2 as described in any one of the preceding embodiments;
S2: subjecting compound 2 to a hydrolysis reaction to prepare compound 1.

The preferred conditions described above may be combined arbitrarily to obtain preferred embodiments of the present disclosure without departing from the general knowledge in the art.

The reagents and starting materials used in the present disclosure are commercially available.

The positive and progressive effects of the present disclosure are as follows: The piperidine ring-containing compound is difficult to form a salt with other acids or bases and has no impurity removal effect. The present disclosure provides benzenesulfonate of a piperidine ring-containing compound, a preparation method therefor, and use thereof. Benzenesulfonate can exist stably, and the content of the isomer impurity represented by formula 1' can be significantly reduced through the steps of preparing the salt form.

### DETAILED DESCRIPTION OF THE PREFERRED EMBODIMENT

The present disclosure is further illustrated by the following examples, which are not intended to limit the present disclosure to the scope of the described examples. Experimental methods without specific conditions indicated in the following examples were performed in accordance with conventional methods and conditions or in accordance with product instructions.

Comparison table of abbreviations:

| Abbreviation | Chinese name |
|---|---|
| IPE | Isopropyl ether |
| ACN | Acetonitrile |
| EtOH | Ethanol |
| IPA | Isopropyl alcohol |
| IPAc | Isopropyl acetate |
| EA | Ethyl acetate |
| PE | Petroleum ether |
| MTBE | Methyl tert-butyl ether |
| TFA | Trifluoroacetic acid |

;
The test methods involved in the following examples were as follows:

High performance liquid chromatography (HPLC)
The detailed parameters were as follows:

| | |
|---|---|
| Instrument model | Agilent 1260 series HPLC |
| Chromatographic column | Ascentis^{®} Express 90 Å C18 150 × 4.6 mm, 2.7 µm |
| Column temperature | 40 °C |
| Mobile phase | A: 0.02% aqueous TFA solution |
| | B: solution of 0.02% TFA in acetonitrile |
| Flow rate | 1.0 mL/min |
| Injection volume | 5 µL |
| Detector | DAD; |
| Detection wavelength | 210 nm |
| Run time | 50 min |
| Diluent | ACN |
| Gradient (time (min)/B%) | 0/10%, 3/30%, 28/45%, 40/95%, 45/95%, 45.1/10%, 50/10% |

;

### Example 1. Preparation of Compound 1

Compound 1 was prepared by referring to the scheme of Example 1 in Patent WO2022022559, and the content of the impurity of prepared compound 1 was about 4%-6%. After compound 1 was purified by column chromatography (EA/PE = 1:10), the content of impurity 1' was reduced to 2.5%-3%, but could not be further reduced.

### Example 2. Preparation of Benzenesulfonate

At 25 °C, compound 1 (2 g, purity: 94.6%, with a content of impurity 1' of 4.21%) was dissolved in 6 V of an IPE/EtOH solution at a volume ratio of 5:1, and a solution of benzenesulfonic acid (335 mg) in IPE (335 mL, 10 wt%) was added to the mixture at a rate of 4 V/h. Then the mixture was stirred for 1 h. After the stirring was completed, a 6 V IPE/ACN solution was added to the mixture at a volume ratio of 15:1, and the resulting mixture was stirred for another 4 days. The resulting mixture was filtered, and the filter cake was dried under vacuum at 30 °C to obtain benzenesulfonate of compound 1 with a purity of 98.9% and a content of impurity 1' of 0.8%.

### Example 3. Preparation of Benzenesulfonate

At 25 °C, compound 1 (2 g, purity: 97.5%, with a content of impurity 1' of 2.32%) was added to ethyl acetate (4 V). The mixture was stirred for 10 min, and then benzenesulfonic acid (0.98 eq) and n-heptane (0.15 V) were added thereto. The mixture was stirred for 9 h and filtered. The filter cake was washed with ethyl acetate (2 V) and dried at 25 °C for 20 h to obtain benzenesulfonate of compound 1 with a purity of 99.0% and a content of impurity 1' of 0.7%.

### Comparative Example 1: Screening of Salt Forms

25 mg of compound 1 prepared in Example 1 was dissolved in 0.5 mL of each solvent, and then an acid or a base (0.55-1.1 eq) was added to the solution at room temperature for a reaction. Various conventional crystallization methods (an anti-solvent method, a solvent volatilization method, a cooling crystallization method, and the like) all failed to induce solid precipitation. The salt formation and impurity removal effects of the products are listed in the table below:
The corresponding situations are as follows:

| Solvent | Acid/base for salt formation | Equivalent | Whether salt can be formed or not | Product form | Impurity removal effect |
|---|---|---|---|---|---|
| MTBE | Benzoic acid | 1.1 | Yes | Oily substance | None |
| Water | Hippuric acid | 1.1 | No | / | None |
| Water | Fumaric acid | 1.1 | No | / | None |
| MTBE | Citric acid | 1.1 | No | / | None |
| Water | Adipic acid | 1.1 | Yes | Oily substance | None |
| Water | Nicotinic acid | 1.1 | Yes | Oily substance | None |
| MTBE | Salicylic acid | 1.1 | Yes | Oily substance | None |
| MTBE | Malic acid | 1.1 | Yes | Oily substance | None |
| MTBE | Diethanolamine | 1.1 | Yes | Oily substance | None |
| IPE/water | Ethylenediamine | 0.55 | Yes | Oily substance | None |
| IPE/water | Calcium hydroxide | 0.55 | Yes | Oily substance | None |
| IPA | Sodium hydroxide | 1.1 | Yes | Oily substance | None |
| MTBE | Aqueous ammonia | 1.1 | Yes | Oily substance | None |
| MTBE | Oxalic acid | 1.1 | Yes | Oily substance | None |

| | | | | | |
|---|---|---|---|---|---|
| Note: The "none" in the table means that it has no impurity removal effect. | | | | | |

As can be seen from the above, compound 1 can form salts with some acids or bases, but the physical forms of these salts are oily substances, which cannot achieve the effect of removing the isomer impurity represented by formula 1'.

## Claims

1. Compound 2:

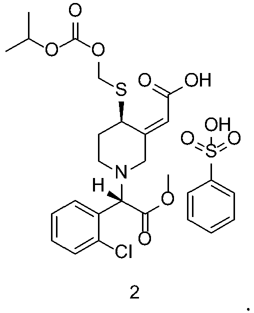

2. A preparation method for compound 2 according to claim 1, wherein comprising the following step: mixing compound 1 with benzenesulfonic acid in a solvent, the solvent is an ether solvent, a mixed solvent of an ether solvent and ethanol, an ester solvent, or an alkane solvent;

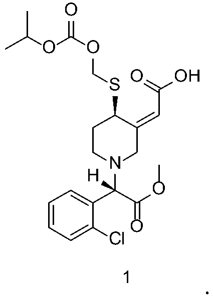

3. The preparation method for compound 2 according to claim 2, wherein comprising the following step: mixing solution 1 containing compound 1 with solution 2 containing benzenesulfonic acid for a reaction, wherein a solvent of solution 1 is an ether solvent, a mixed solvent of an ether solvent and ethanol, or an ester solvent; a solvent of solution 2 is an ether solvent or an alkane solvent.

4. The preparation method for compound 2 according to any one of claims 2-3, wherein one or more of the following conditions are met:
(1) the ether solvent is isopropyl ether;
(2) the ester solvent is ethyl acetate; and
(3) the alkane solvent is n-heptane.

5. The preparation method for compound 2 according to any one of claims 2-3, wherein one or more of the following conditions are met:
(1) a purity of compound 1 is 90%-99.6%;
(2) compound 1 is a crude product of compound 1, and the crude product of compound 1 is a mixture of compound 1 and an isomer impurity represented by formula 1'; a mass ratio of compound 1 to the isomer impurity is (10-100):1; 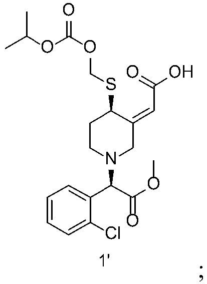
(3) when the solvent is a mixed solvent of an ether solvent and ethanol, a volume ratio of the ether solvent to ethanol is (1-10):1;
(4) a molar ratio of benzenesulfonic acid to compound 1 is (0.3-1):1;
(5) the preparation method for compound 2 further comprises a step of mixing with solvent 3, wherein solvent 3 is a mixed solvent of an ether solvent and acetonitrile or an alkane solvent;
(6) a mixing temperature is 25 °C;
(7) a mixing reaction time is 5-100 h;
(8) a purity of compound 2 prepared by the preparation method for compound 2 is 98.5% or higher; and
(9) a content of impurity 1' in a product prepared by the preparation method for compound 2 is less than 1%.

6. The preparation method for compound 2 according to claim 5, wherein one or more of the following conditions are met:
(1) the purity of compound 1 is 94.6% or 97.5%;
(2) compound 1 is a crude product of compound 1, and the crude product of compound 1 is a mixture of compound 1 and an isomer impurity represented by formula 1'; the mass ratio of compound 1 to the isomer impurity is 23.47:1 or 42.03:1; 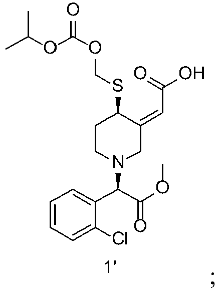
(3) when the solvent is a mixed solvent of an ether solvent and ethanol, the volume ratio of the ether solvent to ethanol is 5:1;
(4) the molar ratio of benzenesulfonic acid to compound 1 is 0.5:1, 0.98:1, or 1:1;
(5) when solvent 3 is an ether solvent and acetonitrile, the volume ratio of the ether solvent to acetonitrile is (13-20):1, such as 15:1;
(6) a mass-to-volume ratio of compound 1 to solvent 3 is 1:(3-45) g/mL, such as 1:6 g/mL, 1:17.85 g/mL, or 1:27 g/mL;
(7) the mixing reaction time is 9.3 h, 22 h, or 73 h;
(8) the purity of compound 2 prepared by the preparation method for compound 2 is 98.9% or 99%; and
(9) the content of impurity 1' in the product prepared by the preparation method for compound 2 is 0.8% or less than 0.1%.

7. The preparation method for compound 2 according to claim 3, wherein one or more of the following conditions are met:
(1) a mass-to-volume ratio of compound 1 to the solvent of solution 1 is 1:(1-10) g/mL, such as 1:2.3 g/mL, 1:4 g/mL, or 1:6 g/mL;
(2) a mass-to-volume ratio of benzenesulfonic acid to the solvent of solution 2 is 1:(0.1-10) g/mL, such as 1:0.15 g/mL, 1:0.7 g/mL, or 1:10 g/mL; and
(3) solution 2 containing benzenesulfonic acid is added to solution 1 containing compound 1, and an addition rate is 1-10 V/h, such as 4 V/h.

8. The preparation method for compound 2 according to claim 2, wherein comprising scheme A or scheme B:
Scheme A: mixing solution 1 containing compound 1 with solution 2 containing benzenesulfonic acid for a reaction, a solvent of solution 1 is a mixed solvent of isopropyl ether and ethanol (e.g., a volume ratio of isopropyl ether to ethanol is 5:1), and a solvent of solution 2 is isopropyl ether; further, after the mixing and reaction, a step of mixing with solvent 3 is further comprised, wherein solvent 3 is a mixed solution of isopropyl ether and acetonitrile (e.g., a volume ratio of isopropyl ether to ethanol is 15:1);
Scheme B: mixing solution 1 containing compound 1 with solution 2 containing benzenesulfonic acid for a reaction, wherein a solvent of solution 1 is ethyl acetate; a solvent of solution 2 is n-heptane.

9. Use of compound 2 according to claim 1 in the preparation of compound 1;

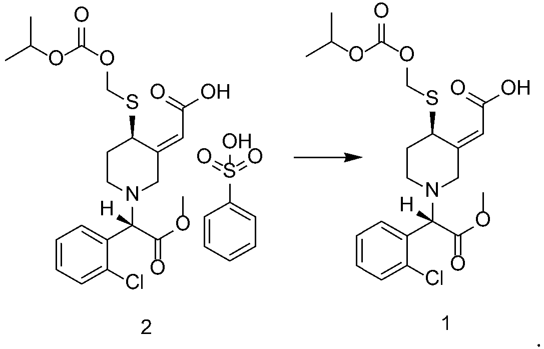

10. A purification method for compound 1, comprising the following steps: 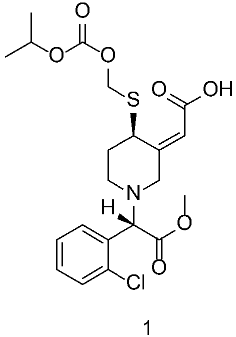
S1: the preparation method for compound 2 according to any one of claims 2-8;
S2: subjecting compound 2 to a hydrolysis reaction to prepare compound 1.
